# EUROPEAN PATENT APPLICATION

(11) **EP 3 042 653 A1**
(43) Date of publication of application: **13.07.2016**
(21) Application number: 14840209.2
(22) Date of filing: 26.08.2014
(51) Int. Cl.: A61K 31/351, A23L 33/10, A61K 31/7048, A61K 36/00, A61K 36/18, A61P 25/20, A61P 43/00

(54) **CLOCK GENE EXPRESSION LEVEL MODIFIER**

(30) Priority: 27.08.2013 JP 2013175147
(71) Applicant: Suntory Holdings Limited, Osaka-shi, Osaka 530-8203 (JP)
(72) Inventor: KASAJIMA, Naoki, Soraku-gun Kyoto 619-0284 (JP); KOMINAMI, Masaru, Soraku-gun Kyoto 619-0284 (JP)
(74) Representative: Vossius & Partner Patentanwälte Rechtsanwälte mbB
(86) International application number: PCT/JP2014/072232
(87) International publication number: WO 2015/029968

(57) **Abstract**

Provided is a clock gene expression level regulator which has a long history of being eaten and is high in safety, easily obtainable and superior in processability.

Specifically provided is a clock gene expression level regulator comprising, as an active component, at least one compound selected from the group consisting of oleuropein, 3,4-DHPEA-EA, derivatives thereof, and salts of the foregoing. The clock gene expression level regulator of the present invention can regulate the expression levels of the *Per1* and *Bmall* genes in antiphase to each other, thereby entraining the expression rhythms of the *Per* and *Bmal* genes to their normal phases.

## Description

### TECHNICAL FIELD

The present invention relates to regulators of the expression levels of clock genes in mammals, and more particularly to regulators of the expression levels of the clock genes *Per* and *Bmal.*

### BACKGROUND ART

Many biological activities exhibit autonomous, periodic changes called biological rhythms (biorhythm). Among them, those rhythms which show a cycle of about 24 hours are called "circadian rhythms."

The sleep rhythm in mammals including humans is normally subject to a circadian rhythm. However, in modem life, the sleep rhythm often fails to follow a circadian rhythm due to various factors including irregular life patterns associated with shiftwork, etc., and long-distance travels via international flights; thus, indispositions and associated diseases, such as jet lag and sleep disorders caused by sleep rhythm disturbance, have been regarded as problematic. In addition, the elderly was found to show a decrease in the synthesis of endogenous factors for establishing a sleep rhythm (*e.g.,* melatonin), so reduction in QOL in the elderly due to sleep rhythm disturbance has also been a concern (Non-patent Literatures 1 and 2). Accordingly, controlling the sleep rhythm to be in a normal state has been recognized as an important challenge.

In order to develop an approach for controlling a sleep rhythm, various attempts have been hitherto made, for example, to administer the particular endogenous hormones, *i.e.,* melatonin and its derivatives. However, hormone administration has been pointed out to be problematic in terms of side effects because it has influence even on other various physiological phenomena than sleep rhythm (Non-patent Literatures 3 and 4).

In recent years, it has been found that circadian rhythms in various biological activities are controlled by a group of genes called clock genes. It was also known that sleep rhythm disorders are associated with alterations of clock genes (Non-patent Literature 5). As specific examples of clock genes, *Per* genes (*Per1, Per2, Per3), Clock* gene, *Bmal* genes *(Bnaal1. Bmal2, Bmal3), Cry* genes (*Cryl*, *Cry2*), and others have been reported. Among them, the *Per* and *Bmal* genes have been demonstrated to be important clock genes. In mammals, the respective types of the *Per* and *Bmal* genes are expressed in different circadian rhythms with phases shifted by about 12 hours; so, *Bmal* expression increases at night whereas *Per* expression increases in daylight (Patent Literature 1). A shift in the phases of the expression rhythms of the *Per* and *Bmal* genes leads to disturbance in circadian rhythms. Therefore, it is considered that control of the sleep rhythm can be achieved if the expression rhythms of the Per and *Bmal* genes can be entrained to their normal phases through regulation of their expression levels. There is a demand for a clock gene expression level regulator which is high in safety and which can effectively entrain the phases of the expression rhythms of the *Per* and *Bmal* genes to their normal state.

### CITATION LIST

### PATENT LITERATURE

Patent Literature 1: WO 2011/122041

### NON-PATENT LITERATURES

Non-patent Literature 1: Vitiello MV., Clin. Cornerstone, 2000; 2 (5): 16-27
Non-patent Literature 2: Mishima K., et al., Chronobiol Int., 2000 May; 17 (3): 419-32
Non-patent Literature 3: Monti JM, Cardinali DP., Biol Signals Recept, 2000, 9: 328-39
Non-patent Literature 4: Reiter RJ., Ann Med, 1998, 30:103-8
Non-patent Literature 5: Ishida, 2009. The Journal of Biochemistry, vol. 81, no. 2, p.75-83

### SUMMARY OF INVENTION

### TECHNICAL PROBLEM

The present invention has as its object to provide a clock gene expression level regulator which is high in safety, daily ingestible, and highly effective, in particular an expression level regulator for the *Per* and *Bmal* genes.

### SOLUTION TO PROBLEM

The present inventors have searched for an effective component from among natural ingredients which have a long history of being eaten and which are high in safety, and as a result, found that oleuropein, a polyphenol extracted from olive, has a capability of regulating the expression levels of the *Per* and *Bmal* clock genes. Further, the inventors found that the metabolite of oleuropein, 3,4-dihydroxyphenylethanol-elenolic acid (hereinafter referred to as "3,4-DHPEA-EA"), also has a capability of regulating the expression levels of said genes. By regulating the expression levels of the *Per* and *Bmal* genes in antiphase to each other, oleuropein and 3,4-DHPEA-EA can entrain the expression rhythms of said genes to their normal phases and eventually can control the sleep rhythm to be in a normal state.

More specifically, the present invention includes, but is not limited to, the following.
[1] A clock gene expression level regulator comprising at least one compound selected from the group consisting of oleuropein, 3,4-dihydroxyphenylethanol-elenolic acid, derivatives thereof, and salts of the foregoing.
[2] The clock gene expression level regulator as set forth in [1], comprising at least one compound selected from the group consisting of oleuropein, derivatives thereof, and salts of the foregoing.
[3] The clock gene expression level regulator as set forth in [1], comprising at least one compound selected from the group consisting of 3,4-dihydroxyphenylethanol-elenolic acid, derivatives thereof, and salts of the foregoing.
[4] The clock gene expression level regulator as set forth in any one of [1] to [3], wherein the clock gene expression level regulator regulates the expression levels of *Per* and *Bmal* genes in antiphase to each other to thereby entrain the expression rhythms of the *Per* and *Bmal* genes to their normal phases.
[5] The clock gene expression level regulator as set forth in [4], wherein the *Per* gene is *Perl* gene, and the *Bmal* gene is *Bmal1* gene.
[6] A clock gene expression level regulator comprising an olive extract.
[7] The clock gene expression level regulator as set forth in [6], wherein the olive extract comprises at least 30% by weight of an olive-derived polyphenol.
[8] The clock gene expression level regulator as set forth in [7], wherein the olive-derived polyphenol comprises at least one compound selected from the group consisting of oleuropein, 3,4-dihydroxyphenylethanol-elenolic acid, derivatives thereof, and salts of the foregoing.
[9] The clock gene expression level regulator as set forth in any one of [6] to [8], wherein the olive extract is extracted from olive leaves with water, ethanol, or a mixture thereof.
[10] A food or beverage having added thereto the clock gene expression level regulator as set forth in any one of [1] to [9].
[11] A pharmaceutical composition for preventing, alleviating, or treating indispositions or diseases caused by sleep rhythm disturbance, the pharmaceutical composition comprising at least one compound selected from the group consisting of oleuropein, 3,4-dihydroxyphenylethanol-elenolic acid, derivatives thereof, and salts of the foregoing.
[12] A method for preparing a food, beverage or pharmaceutical composition having sleep rhythm-controlling activity, the method comprising the step of incorporating at least one compound selected from the group consisting of oleuropein, 3,4-dihydroxyphenylethanol-elenolic acid, derivatives thereof, and salts of the foregoing.
[13] Use of at least one compound selected from the group consisting of oleuropein, 3,4-dihydroxyphenylethanol-elenolic acid, derivatives thereof, and salts of the foregoing, in the preparation of a food, beverage or pharmaceutical composition having sleep rhythm-controlling activity.

### ADVANTAGEOUS EFFECTS OF INVENTION

The clock gene expression level regulator of the present invention, which comprises oleuropein and/or 3,4-dihydroxyphenylethanol-elenolic acid (hereinafter also referred to as "3,4-DHPEA-EA") as an active component, can regulate the expression levels of two types of *Per* and *Bmal* genes in antiphase to each other, thereby making it possible to entrain the expression rhythms of said genes to their normal phases and to control the sleep rhythm effectively. Since the inventive clock gene expression level regulator comprises, as an active component, oleuropein and/or 3,4-DHPEA-EA which are present in olive, this is a regulator having a long history of being eaten, little side effects, and high safety.

### BRIEF DESCRIPTION OF DRAWINGS

FIG. 1 shows the genetic expression level of the *Bmal1* clock gene in the liver of SD rats treated with an oleuropein-rich olive extract.
FIG. 2 shows the genetic expression level of the *Per1* clock gene in the liver of SD rats treated with an oleuropein-rich olive extract.
FIG. 3 shows the genetic expression level of the *Bmal1* clock gene in the human liver cancer-derived cell line, HepG2, treated with 3,4-DHPEA-EA.
FIG. 4 shows the genetic expression level of the *Per1* clock gene in the human liver cancer-derived cell line, HepG2, treated with 3,4-DHPEA-EA.

### DESCRIPTION OF EMBODIMENTS

### <Clock gene expression level regulator>

The clock gene expression level regulator of the present invention, which comprises oleuropein and/or 3,4-DHPEA-EA as an active component, can regulate the expression levels of the *Per* and *Bmal* clock genes in antiphase to each other, thereby making it possible to entrain the expression rhythms of the *Per* and *Bmal* genes to their normal phases and to control the sleep rhythm to be in a normal state. Therefore, the inventive clock gene expression level regulator can be effectively used in the prevention, alleviation, treatment, etc. of indispositions and diseases caused by sleep rhythm disturbance, such as sleep disorders and jet lag.

### <Per and Bmal genes>

Since the clock gene expression level regulator of the present invention comprises oleuropein and/or 3,4-DHPEA-E as an active component, the inventive regulator can regulate the expression levels of the certain clock genes, in particular regulate the expression levels of the *Per* and *Bmal* genes in antiphase to each other.

Clock genes refer to a group of genes which play a role in controlling circadian rhythms, and are defined as genes which affect activity rhythms when they are altered (Ishida, 2009, The Journal of Biochemistry, vol. 81, no. 2, p.75-83). Particularly principal factors are *Period* (*Per*) genes and *Bmal* genes.

In mammals, the *Period* gene family consisting of *period1 (per1), period2 (per2)* and *period3 (per3)* is known to function as clock genes. *Smal1,* a member of the *Bmal* gene family, is also known to function as a clock gene. The *Bmall* gene product, *BMAL1,* forms a heterodimer with *CLOCK* to stimulate the transcription of *Period* genes, whereas the *Period* gene product, *PERIOD,* forms a heterodimer with *CRYPTOCHROME* to suppress the activities of *BMAL1* and *CLOCK.*

In mammals, the respective types of the *Per* and *Bmal* genes are expressed in different circadian rhythms with phases shifted by about 12 hours; so, *Bmal* expression increases at night whereas *Per* expression increases in daylight. These phases are normal for the expression rhythms of the *Per* and *Bmal* genes. A shift in the phases of the expression rhythms of the *Per* and *Bmal* genes leads to disturbance in circadian rhythms such as sleep rhythm.

The clock gene expression level regulator of the present invention can regulate the expression levels of the *Per* and *Bmal* genes in antiphase to each other, thereby entraining the expression rhythms of said genes to their normal phases.

### <Analysis of Per and Bmal gene expression levels>

The expression levels of the *Per* and *Bmal* genes regulated by the inventive clock gene expression level regulator can be evaluated using a given method known to those skilled in the art.

To perform evaluation in living organisms, a test animal such as mouse, rat, guinea pig, rabbit, dog, cat, horse, monkey, or human can be used depending on the purpose. A control group is not treated with a test substance while a test group is treated with a test substance, and both of the groups are fed for a certain period of time. Any mode of administration, such as oral, topical or enteral administration, can be selected depending on the purpose. After the feeding period, biological samples are collected from each of the control and test groups. Any biological sample can be put to use in the analysis of gene expression as long as the sample contains cells; for example, samples of skin, liver, and hair containing hair follicle cells, as well as blood, saliva and other samples can be used. Total RNA is extracted from the collected samples according to a conventional method and used to analyze clock gene expression.

To perform evaluation in cultured cells, any cultured cell line can be used, but liver-derived cell lines such as HepG2 can be used advantageously. The cells from a control group are not treated with a test substance while the cells from a test group are treated with a test substance, and both of the cells are incubated for a certain period of time. After the incubation period, the cells of the respective groups are harvested, and total RNA is extracted from these cells according to a conventional method and used to analyze clock gene expression.

In the process of performing the evaluations in living organisms or cultured cells, an analysis of clock gene expression can be made advantageously by such a method as Northern blotting analysis using a certain region of a *Per* or *Bmal* gene sequence as a probe, realtime RT-PCR analysis, or DNA microarray-based gene expression analysis.

### <Compound>

The clock gene expression level regulator of the present invention comprises oleuropein and/or 3,4-DHPEA-EA as an active component.

In the present invention, not only oleuropein and 3,4-DHPEA-EA but also derivatives thereof and any salt forms of the foregoing can be used. The derivatives of oleuropein or 3,4-DHPEA-EA include a group of compounds obtained by chemical modification of the hydroxyl group or the like of oleuropein or 3,4-DHPEA-EA. and can be exemplified by esters and glycosides of oleuropein or 3,4-DHPEA-EA.

### <Oleuropein>

Oleuropein (CAS No. 32619-42-4) is one of the polyphenol components abundant in olive. Oleuropein has the structure shown below:

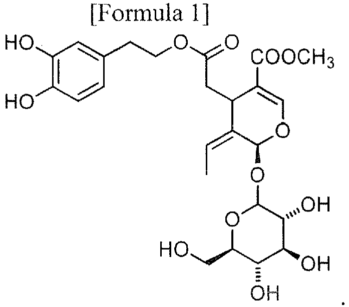

### <3,4-DHPEA-EA>

3,4-Dihydroxyphenylethanol-elenolic acid (3,4-DHPEA-EA) is one of the principal polyphenols contained in olive oil, in particular extra-virgin olive oil.

It was reported that 3,4-DHPEA-EA is produced by the metabolism of oleuropein through the activity of an endogenous *E. coli* β-glucosidase *(*J Biolechnol., 2004 Jul 1; 111 (1):67-77).

3,4-DHPEA-EA can be represented by the structure shown below: 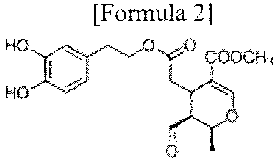 or

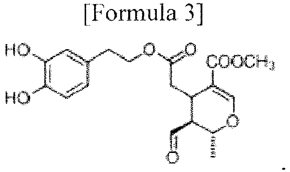

3,4-DHPEA-EA, which is obtained by hydrolysis of oleuropein by a β-glucosidase, is a mixture of diastereomers which differ in the configuration of the methyl group at position *9* (Natural Products Research, 19(2), 105-109 (2005); and Journal ofAgricultural and Food Chemistry, 49, 3198-3203 (2001))

### <Clock gene expression level regulator>

In the clock gene expression level regulator of the present invention, plant ingredients like olive, which are rich in oleuropein and/or 3,4-DHPEA-EA, can be directly used as an active component. Also, an olive extract containing a high concentration of oleuropein and/or 3,4-DHPEA-EA, which is extracted from plant ingredients such as olive, may be used as an active component of the inventive clock gene expression level regulator. Isolated oleuropein or isolated 3,4-DHPEA-EA from plant ingredients such as olive may be used as an active component of the inventive regulator.

In the case where plant ingredients rich in oleuropein and/or 3,4-DHPEA-EA are directly used as the inventive clock gene expression level regulator, leaves, fruits, seeds, stems and other portions of olive, for example, can be used raw or in a dried state achieved by freeze-drying or other means. In the case of using olive leaves, a mixture of olive leaves with leaves of other plant species, such as tea leaves, may be used. An olive oil obtained by pressing olive fruits may also be used. The olive oil to be used can be a commercially available one, or can be prepared by a known method.

Also, isolated oleuropein or isolated 3,4-DHPEA-EA can be obtained from plant ingredients such as olive by the method described later.

The amount of oleuropein and/or 3,4-DHPEA-EA contained as an active component in the clock gene expression level regulator of the present invention can be suitably determined in consideration of effectiveness, but said active component is present in the inventive regulator in an amount of preferably from 0.01 to 60% by weight, more preferably from 0.1 to 50% by weight, still more preferably from 1 to 40% by weight. In the case of using oleuropein as an active component, this component is present in the inventive regulator in an amount of preferably from 0.01 to 60% by weight, more preferably from 0.1 to 50% by weight, still more preferably from 1 to 40% by weight. In the case of using 3.4-DHPEA-EA as an active component, this component is present in the inventive regulator in an amount of preferably from 0.01 to 60% by weight, more preferably from 0.1 to 50% by weight, still more preferably from 1 to 40% by weight. Depending on the purpose, a combination of oleuropein and 3,4-DHPEA-EA may be used as an active component of the inventive clock gene expression level regulator. In this case, oleuropein and 3,4-DHPEA-EA, taken together, are present in the inventive regulator in a total amount of preferably from 0.01 to 60% by weight, more preferably from 0.1 to 50% by weight, still more preferably from 1 to 40% by weight.

### <Olive>

In the clock gene expression level regulator of the present invention, plant ingredients rich in oleuropein and/or 3,4-DHPEA-EA, such as leaves, fruits, seeds, stems and other portions of olive, can be used raw, or an olive extract containing a high concentration of oleuropein and/or 3,4-DHPEA-EA, or isolated oleuropein or 3,4-DHPEA-EA, which are obtained from such plant ingredients as olive, can also be used. Any variety of olive can be used; for example, olives of the following varieties can be used advantageously: Manzanillo, Lucca, Nevadillo blanco, Mission, Picual, Arbequina, Hojiblanca, Cornicabra. Gordal, Moraiolo, Frantoio, Coratina, and Leccino.

As the plant ingredients used for preparing an olive extract containing a high concentration of oleuropein and/or 3,4-DHPEA-EA, or isolated oleuropein or 3,4-DHPEA-EA, any portions of plants can be used, such as leaves, fruits, seeds, and stems of olive, with plant leaves being preferably used. The plant ingredients can be used raw or in a dried state achieved by freeze-drying or other means. Leftover residues after pressing oil from olive fruits may also be used raw or in a dried state.

### <Olive extract>

To prepare an olive extract containing a high concentration of oleuropein and/or 3,4-DHPEA-EA, any method can be used as along as an extract rich in an olive-derived polyphenol can be obtained by said method. As referred to herein, the olive-derived polyphenol is a polyphenol contained in olive, and refers to the one having a hydroxyrtyrosol backbone as part of its structure, the one having a tyrosol backbone as part of its structure, the one having a coffeic acid backbone as part of its structure, the one having a p-coumaric acid backbone as part of its structure, or a mixture thereof. Examples of the olive-derived polyphenol having a hydroxytyrosol backbone as part of its structure include hydroxytyrosol, and precursors, metabolites and the like thereof. Examples of the olive-derived polyphenol having a tyrosol backbone as part of its structure include tyrosol, and precursors, metabolites and the like thereof. Examples of the olive-derived polyphenol having a coffeic acid backbone as part of its structure include coffeic acid, and precursors, metabolites and the like thereof. Examples of the olive-derived polyphenol having a p-coumaric acid backbone as part of its structure include p-coumaric acid, and precursors, metabolites and the like thereof. The olive-derived polyphenol contains oleuropein in an amount of preferably from 0.01 to 60% by weight, more preferably from 0.1 to 50% by weight, still more preferably from 1 to 40% by weight.

The olive extract containing a high concentration of oleuropcin and/or 3,4-DHPEA-EA according to the present invention can be prepared by a method in which an extract containing an olive-derived polyphenol in abundance, preferably in an amount of 30% by weight or more, can be obtained. More specifically, the olive extract containing a high concentration of oleuropein and/or 3,4-DHPEA-EA can be prepared, for example, according to the procedure described below.
(1) First, there is prepared an aqueous olive extract. The aqueous olive extract can be obtained using this plant ingredient by an extraction method commonly used in plant extraction. For example, the aqueous olive extract can be obtained by providing olive leaves in a raw or dried state and subjecting them, as they are or after crushed by a coarse grinder, to extraction with an aqueous solvent. A commercially available olive leaf extract (e.g., produced by Eisai Food & Chemical Co., Ltd.) may also be used.
(2) Next, the aqueous olive extract is introduced into a column charged with an adsorption resin and eluted with water, ethanol or a mixture thereof to obtain a concentrated fraction of oleuropein and/or 3,4-DHPEA-EA. As the adsorption resin, any resin can be used as long as it is capable of adsorbing oleuropein and/or 3,4-DHPEA-EA; for example, the synthetic polymer resin MCI-GEL CHP20 (produced by Mitsubishi Chemical Corporation) can be advantageously used. As for the elution with water, ethanol or a mixture thereof, those skilled in the art could determine the elution conditions, as appropriate, depending on the properties of the aqueous olive extract and adsorption resin to be used. For example, the aqueous olive extract can be subjected to stepwise elution with water, an aqueous 20.0% ethanol solution, an aqueous 30.0% ethanol solution, an aqueous 32.5% ethanol solution, an aqueous 35% ethanol solution, and an aqueous 37.5% ethanol solution, and a fraction obtained using the aqueous 37.5% ethanol solution can be used in this invention as the olive extract containing a high concentration of oleuropein and/or 3,4-DHPEA-EA.

In the olive extract containing a high concentration of oleuropein and/or 3,4-DHPEA-EA, oleuropein and/or 3.4-DHPEA-EA is present at a concentration of preferably from 0.01 to 60% by weight, more preferably from 0.1 to 50% by weight, still more preferably from 1 to 40% by weight.

### <Isolated oleuropein>

Isolated oleuropein can be prepared, for example, according to the procedure described below.
(1) The olive extract prepared by following the procedure described in the preceding paragraph is dissolved in a solvent such as an aqueous acetonitrile solution, and the resulting solution is passed through a filter and then subjected to preparative high-performance chromatography or the like to obtain an oleuropein fraction.
(2) The resulting oleuropein fraction is dried by freeze-drying or the like to obtain an isolated oleuropein powder.

### <Isolated 3,4-DHPEA-EA>

Isolated 3,4-DHPEA-EA can be prepared, for example, according to the procedure described below.
(1) To the isolated oleuropein prepared by following the above-described procedure, β-glucosidase is added, and the mixture is reacted.
(2) After completion of the reaction, the reaction mixture is concentrated under reduced pressure to obtain a coarse fraction of 3.4-DHPEA-EA.
(3) The resulting coarse fraction of 3,4-DHPEA-EA is purified by preparative high-performance liquid chromatography or the like to obtain 3,4-DHPEA-EA.
(4) The resulting 3,4-DHPEA-EA is dried by freeze-drying or the like to obtain isolated 3,4-DHPEA-EA.

### <Food or beverage having added thereto a clock gene expression level regulator>

The clock gene expression level regulator of the present invention can be added to a food or beverage. In other words, this invention provides a food or beverage having added thereto a clock gene expression level regulator. For example, the inventive food or beverage can be used in any orally ingestible form, including: supplements comprising oleuropein and/or 3,4-DHPEA-EA as an active component; functional foods produced by incorporating oleuropein and/or 5,4-DHPEA-EA in a common food or beverage to impart clock gene expression regulatory activity to the food or beverage (including health foods such as dietary supplements, foods with nutrient function claims, foods for special dietary uses, and foods for specified health uses, as well as animal supplements); and animal feeds. The inventive food or beverage having added thereto a clock gene expression level regulator can include, in its package, container or instructions for use, the information about the type and intended use of its active component, its efficacies and effects on e.g., regulation of clock gene expression and improvement of sleep rhythm, and/or its ingestion method.

The food or beverage having added thereto a clock gene expression level regulator according to the present invention can be provided in any form, including: beverages such as juice, milk, coffee beverage and tea beverage; liquid foods such as soup; paste foods such as yoghurt; semi-solid foods such as jelly and gummy candy; solid foods such as cookie and chewing gum; and fat-containing foods such as dressing and mayonnaise. The form of the food or beverage is not particularly limited; for example, the food or beverage can be prepared in any form, including: solid forms such as powder, granule and tablet; liquid forms such as solution, emulsion and dispersion; or semi-solid forms such as paste. The specific dosage form of the food or beverage can be exemplified by powder, granule, fine granule, tablet, pill, troche, capsule (including soft and hard capsules), chewable tablet, and solution. Also, tea or other leaves are mixed with olive leaves, and oleuropein and/or 3,4-DHPEA-EA is added to the mixture, whereby a food or beverage with strengthened clock gene expression regulating capability can be prepared.

The food or beverage having added thereto a clock gene expression level regulator according to the present invention can be mixed if necessary with any other additive, including: minerals; vitamins such as vitamin E, vitamin C and vitamin A; nutritional components; flavors; and pigments, as long as such an additive does not impair the effects of oleuropein and/or 3,4-DHPEA-EA, or in other words, as long as mixing such an additive with oleuropein and/or 3,4-DHPEA-EA does not produce any unfavorable interaction. As such an additive, any of those commonly used in foods and beverages can be used. Also the inventive food or beverage having added thereto a clock gene expression level regulator may be mixed with any other physiologically active component as long as such a component does not impair the effects of oleuropein and/or 3,4-DHPEA-EA.

The food or beverage having added thereto a clock gene expression level regulator according to the present invention comprises oleuropein and/or 3,4-DHPEA-EA in an amount of preferably from 0.001 to 20% by weight, more preferably from 0.01 to 15% by weight, still more preferably from 0.1 to 10% by weight. In the case of using oleuropein, the inventive food or beverage comprises this component in an amount of preferably from 0.001 to 20% by weight, more preferably from 0.01 to 15% by weight, still more preferably from 0.1 to 10% by weight. In the case of using 3,4-DHPEA-EA, the inventive food or beverage comprises this component in an amount of preferably from 0.001 to 20% by weight, more preferably from 0.01 to 15% by weight, still more preferably from 0.1 to 10% by weight.

### <Pharmaceutical composition for preventing, alleviating, or treating indispositions or diseases caused by sleep rhythm disturbance>

The pharmaceutical composition of the present invention, which comprises oleuropein and/or 3,4-DHPEA-EA as an active component, can be used for preventing, alleviating, or treating indispositions or diseases caused by sleep rhythm disturbance, such as sleep disorders caused by irregular life patterns and jet lag due to travel by airplane.

The pharmaceutical composition of the present invention can be formulated with a pharmaceutically acceptable carrier, diluent, excipient or the like by following a common method depending on the purpose. Examples of the diluent and carrier include liquid diluents such as water, ethanol, propylene glycol and glycerin, and solid diluents or excipients such as glucose, sucrose, dextrin, cyclodextrin and gum arabic. Also, an emulsifier, isotonizing agent, buffer, solubilizer, preservative, stabilizer, anti-oxidant and/or the like, which are commonly used in pharmaceutical formulation, may be added as appropriate.

The pharmaceutical composition of the present invention can be mixed if necessary with any other additive, including: minerals; vitamins such as vitamin E, vitamin C and vitamin A; nutritional components; flavors; and pigments, as long as such an additive does not impair the effects of oleuropein and/or 3,4-DHPEA-EA, or in other words, as long as mixing such an additive with oleuropein and/or 3,4-DHPEA-EA does not produce any unfavorable interaction. As such an additive, any of those commonly used in pharmaceutical products can be used. The inventive pharmaceutical composition can also be mixed with any other physiologically active component as long as such a component does not impair the effects of oleuropein and/or 3,4-DHPEA-EA.

The form of the pharmaceutical composition of the present invention is not particularly limited; for example, the pharmaceutical composition can be prepared in any form, including: solid forms such as powder, granule and tablet; liquid forms such as solution, emulsion and dispersion; or semi-solid forms such as paste. The specific dosage form of the pharmaceutical composition can be exemplified by powder, granule, fine granule, tablet, pill, troche, capsule (including soft and hard capsules), chewable tablet, and solution.

The pharmaceutical composition of the present invention comprises oleuropein and/or 3,4-DHPEA-EA in an amount of preferably from 0.1 to 50% by weight, more preferably from 1 to 20% by weight. In the case of using oleuropein, the inventive pharmaceutical composition comprises this component in an amount of preferably from 0.1 to 50% by weight, more preferably from 1 to 20% by weight. In the case of using 3,4-DHPEA-EA, the inventive pharmaceutical composition comprises this component in an amount of preferably from 0.1 to 50% by weight, more preferably from 1 to 20% by weight.

The dose and dosage form of the pharmaceutical composition of the present invention can be selected as appropriate depending on the subject to be treated, the pathological condition and its progress, and other requirements. For example, if the pharmaceutical composition is orally administered for the purpose of obtaining a regulatory effect on clock gene expression in a human (adult) subject, it is generally advisable to administer the pharmaceutical composition consecutively about one to three times a day so as to give a daily dose of oleuropein and/or 3,4-DHPEA-EA of from about 0.01 to 500 mg, preferably from about 0.1 to 300 mg, more preferably from about 10 to 200 mg. As for the case of using oleuropein, if the pharmaceutical composition is orally administered for the purpose of obtaining a regulatory effect on clock gene expression in a human (adult) subject, it is advisable to administer the pharmaceutical composition consecutively about one to three times a day so as to give a daily dose of oleuropein of from about 0.01 to 500 mg, preferably from about 0.1 to 300 mg, more preferably from about 1 to 200 mg. As for the case of using 3,4-DHPEA-EA, if the pharmaceutical composition is orally administered for the purpose of obtaining a regulatory effect on clock gene expression in a human (adult) subject, it is advisable to administer the pharmaceutical composition consecutively about one to three times a day so as to give a daily dose of 3,4-DHPEA-EA of from about 0.01 to 500 mg, preferably from about 0.1 to 300 mg, more preferably from about 1 to 200 mg. As for the case of using oleuropein and 3,4-DHPEA-EA in combination, if the pharmaceutical composition is orally administered for the purpose of obtaining a regulatory effect on clock gene expression in a human (adult) subject, it is advisable to administer the pharmaceutical composition consecutively about one to three times a day so as to give a total daily dose of oleuropein and 3,4-DHPEA-EA of from about 0.01 to 500 mg, preferably from about 0.1 to 300 mg, more preferably from about 1 to 200 mg.

### <Preparation of a food, beverage, or pharmaceutical composition>

The clock gene expression level regulator of the present invention, which comprises oleuropein and/or 3,4-DHPEA-EA as an active component, can regulate the expression levels of the *Per* and *Bmal* clock genes in antiphase to each other. The inventive regulator can be provided in the form of a food, beverage, or pharmaceutical composition.

In other words, from another viewpoint, the present invention provides a method for preparing a food, beverage or pharmaceutical composition having a capability of regulating clock gene expression levels, the method comprising the step of incorporating oleuropein and/or 3,4-DHPEA-EA. From still another viewpoint, this invention provides use of oleuropein and/or 3,4-DHPEA-EA in the preparation of a food, beverage or pharmaceutical composition having a capability of regulating clock gene expression levels.

Hereunder, the present invention will be described more specifically on the basis of working examples. However, this invention is not limited to these working examples.

### EXAMPLE 1

### Test Example 1: Regulatory effect of isolated oleuropein on clock gene expression levels

The regulatory effect of isolated oleuropein on clock gene expression levels was evaluated according to the procedure described below.

### <Test sample>

The isolated oleuropein used was obtained by following steps (1) and (2) below.
(1) An olive leaf extract (102 g; produced by Eisai Food & Chemical Co., Ltd.) was dissolved in 24 times its volume of water, and the solution was introduced into a column charged with the synthetic polymer resin MCI-GEL CHP20 (1.2 L; produced by Mitsubishi Chemical Corporation). Then, the column was eluted with water (1.2 L), an aqueous 20.0% ethanol solution (1.2 L), an aqueous 30.0% ethanol solution (1.2 L), an aqueous 32.5% ethanol solution (1.2 L), an aqueous 35% ethanol solution (1.2L), and an aqueous 37.5% ethanol solution (1.2 L) to obtain a concentrated fraction of oleuropein (42 g).
(2) Next, the thus-obtained concentrated oleuropein fraction was dissolved in an aqueous 30% acetonitrile solution to give a concentration of 45% (w/v), and the resulting solution was passed through a 0.45 µm membrane filter and subjected to preparative high-performance chromatography to isolate oleuropein (27 g).

### (Preparative HPLC conditions)

Detector: 295 nm
Column: Develosil ODS-HG-15/30 (ϕ5.0×500 mm; produced by Nomura Chemical Co., Ltd.)

### <Evaluation method>

Seven-week-old male SD rats were habituated for one week and then classified into a test food group (isolated oleuropein group) and a control group (each group consisting of 6 animals). Next, the isolated oleuropein was dissolved in distilled water (produced by Otsuka Pharmaceutical Co., Ltd.), and the solution was orally administered forcibly to the test food group at a dose of 200 mg/5 mL/kg once daily for 5 days. The control food group was orally fed forcibly with distilled water (produced by Otsuka Pharmaceutical Co., Ltd.) at a dose of 5 mL/kg once daily for 5 days. Four hours after the last treatment, liver sampling was done.

From the liver samples collected, total RNA was extracted according to a conventional method, and used to analyze the expression levels of the *Bmal1* and *Per1* clock genes by quantitative PCR (with Applied Biosystems 7900HT Fast Real-Time PCR System).

### <Results>

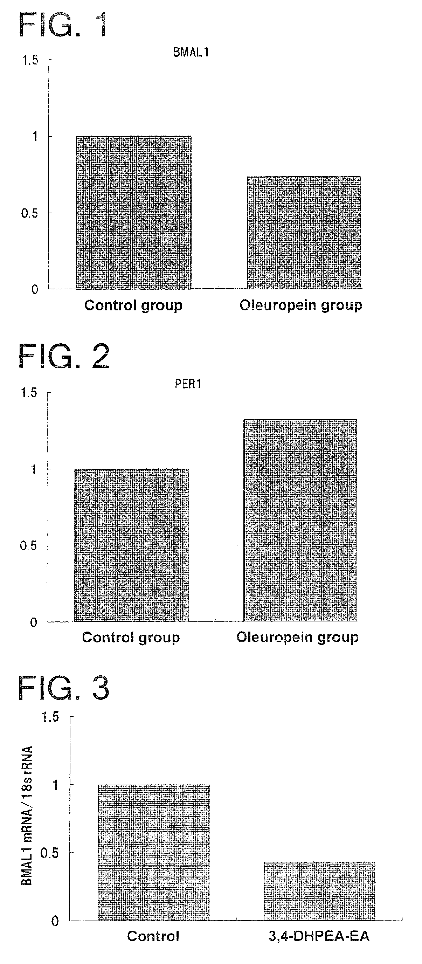

FIG. 1 shows the expression level of the *Bal1* gene, and FIG. 2 shows the expression level of the *Per1* gene. As compared to the control group, the isolated oleuropein group showed a significant decrease in *Bmal1* expression and a significant increase in *Per1* expression. As described in the foregoing, it has been found that the expression levels of *Bmal1* and *Per1* are in antiphase to each other in a normal circadian rhythm. In this analysis, it was confirmed that the *Bmal1* and *Per1* expressions in the isolated oleuropein group were varied in antiphase to each other. More specifically, isolated oleuropein reduced *Bmal1* expression while increasing *Per1* expression. That is to say, oleuropein was found to be capable of regulating the expressions of clock genes to their normal patterns, thereby helping to entrain shifted phases of the gene expressions to their normal patterns.

### EXAMPLE 2

### Test Example 2: Regulatory effect of isolated 3,4-DHPEA-EA on clock gene expression levels

The regulatory effect of isolated 3,4-DHPEA-EA on clock gene expression levels was evaluated according to the procedure described below.

### <Test sample>

The isolated 3,4-DHPEA-EA used was obtained by following the step described below.

A β-glucosidase (produced by Sigma) was added to oleuropein (100 mg), and the mixture was reacted in a buffer solution with a pH of 6.86 at 37°C for 15 hours. After completion of the reaction, the reaction mixture was concentrated under reduced pressure to obtain a coarse fraction of 3,4-DHPEA-EA. The resulting coarse fraction of 3,4-DHPEA-EA was subjected to preparative high-performance liquid chromatography to isolate 9.0 mg of 3,4-DHPEA-EA.

### (Preparative HPLC conditions)

Detector: 295 nm
Column: Develosil ODS-HG-15/30 (ϕ5.0×500 mm; produced by Nomura Chemical Co., Ltd.)
Solvent: Aqueous 45% acetonitrile solution
Column temperature: Room temperature
Flow rate: 30 mL/min

The nuclear magnetic resonance spectrum data of the obtained 3,4-DHPEA-EA sample was shown below.

**[Table 1]**

| ¹H-, ¹³C-NMR data for 3,4-DHPEA EA | | | | |
|---|---|---|---|---|
| Position | 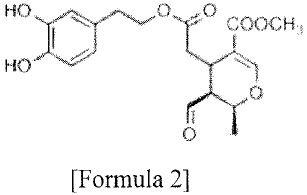 | | 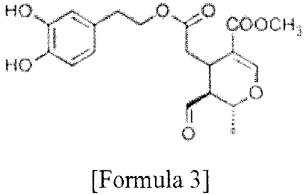 | |
| | ¹H | ¹³C | ¹H | ¹³C |
| 1 | 9.527 (1H, s) | 199.9 | 9 525(1H, s) | 202.2 |
| 3 | 7.56 (1H, s) | 157.2 | 7.59(1H, s) | 156.0 |
| 4 | | 106.5 | | 106.4 |
| 5 | 3.40 (1H, m | 28.1 | 3.36 (1H, m) | 26.9 |
| 6 | 2.21 (1H, dd, J=11, 16 Hz) | 38.9 | 2.36 (1H, dd, J=10.17Hz) | 37.1 |
| | 2.88 (1H, m) | | 2.88 (1H, m) | |
| 7 | | 171.8 | | 171.1 |
| 8 | 2.63 (1H, m) | 50.9 | 2.63 (1H, m) | 54.4 |
| 9 | 4.16 (1H, m) | 69.7 | 4.47 (1H, m) | 70.8 |
| 10 | 1.56 (1H, d, J=7 Hz) | 17.9 | 1.45 (1H, d, J=7 Hz) | 19.3 |
| -OMe | 3.77 (3H, s) | 51.8 | 3.75 (3H, s) | 51.7 |
| 1' | 4.23 (1H, m) | 65.4 | 4.23 (1H, m) | 65.3 |
| | 4.32 (1H, m) | | 437 (1H, m) | |
| 2" | 2.83 (1H, m) | 34.2 | 2.83 (1H, m) | 34.2 |
| 3' | 6.33 (1H, d, J=2 Hz) | 130.4 | 6.78 (1H, d, J=1.5Hz) | 130.3 |
| 4' | | 115.2 | | 115.2 |
| 5' | | 143.1 | | 143.0 |
| 6' | | 143.3 | | 143.4 |
| *T* | 6.86 (1H,d, J=8 Hz) | 116.5 | 6.80 (1H,d, J=8 Hz) | 116.3 |
| 8' | 6.63 (1H, br d, J=8 Hz) | 121.2 | 6.63 (1H, br d, J=8 Hz) | 121.3 |

| | | | | |
|---|---|---|---|---|
| Measured in CDCl₃ | | | | |

### <Evaluation method>

Cultured cells (human liver cancer-derived cell line HepG2) were seeded at 2.5 × 10³ cells/cm² in a T-75 flask (BD Falcon^{™}), and medium replacement (RPMI1640, 10% FBS) was performed every three days. The cells reaching 80% confluence were passaged at a concentration of 4.2 × 10⁴ cells/mL in a 6-well plate (BD Falcon^{$},), and in order to entrain the phases of the circadian rhythms of the cells, the medium was replaced with a low-serum medium (RPMI1640, 2% FBS) after three days. Three days after the replacement with a low-serum medium, the cells were subjected to testing. A solution of the test substance in DMSO (50 µg/µL) was prepared and added to the 6-well plate to give a final concentration of the test substance of 50 µg/mL. As a control, DMSO was added to the 6-well plate (to give a final concentration of 0.1 %).

Eight hours after the addition of the test substance, the cells were harvested, and mRNA was extracted according to a conventional method. The extracted mRNA was used to analyze the expression levels of the *Bmall* and *Per1* clock genes by qPCR (with Applied Biosystems 7900HT Fast Real-Time PCR System).

### <Results>

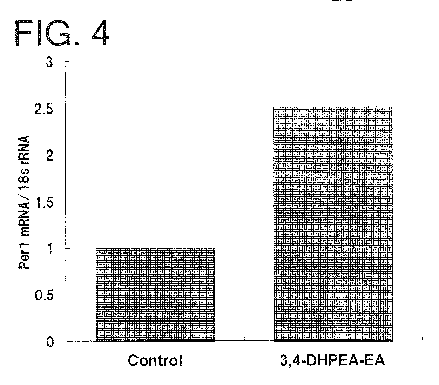

FIG. 3 shows the expression level of the *Bma1 l*gene, and FIG. 4 shows the expression level of the *Per1* gene. As described in the foregoing, it has been found that the expression levels of *Bmall* and *Per1* are in antiphase to each other in a normal circadian rhythm. In this analysis, it was confirmed that when 3,4-DHPEA-EA was added, the expression levels of *Bmall* and *Per1* were modulated in antiphase to each other. More specifically, 3,4-DHPEA-EA reduced *Bmall* expression while increasing *Per1* expression. That is to say, 3,4-DHPEA-EA was found to be capable of regulating the expressions of clock genes to their normal patterns, thereby helping to bring shifted phases of the gene expressions back to normal.

### INDUSTRIAL APPLICABILITY

The clock gene expression level regulator of the present invention, which comprises oleuropein and/or 3,4-DHPEA-EA as an active component, can effectively regulate the expression levels of clock genes. Therefore, the inventive clock gene expression level regulator can be effectively used for preventing, alleviating, or treating indispositions or diseases caused by sleep rhythm disturbance, such as sleep disorders and jet lag.

## Claims

1. A clock gene expression level regulator comprising at least one compound selected from the group consisting of oleuropein, 3,4-dihydroxyphenylethanol-elenolic acid, derivatives thereof, and salts of the foregoing.

2. The clock gene expression level regulator as set forth in claim 1, comprising at least one compound selected from the group consisting of oleuropein, derivatives thereof, and salts of the foregoing.

3. The clock gene expression level regulator as set forth in claim 1, comprising at least one compound selected from the group consisting of 3.4-dihydroxyphenylethanol-elenolic acid, derivatives thereof, and salts of the foregoing.

4. The clock gene expression level regulator as set forth in any one of claims 1 to 3, wherein the clock gene expression level regulator regulates the expression levels of *Per* and *Bmal* genes in antiphase to each other to thereby entrain the expression rhythms of the *Per* and *Bmal* genes to their normal phases.

5. The clock gene expression level regulator as set forth in claim 4, wherein the *Per* gene is *Per1* gene, and the *Bmal* gene is *Bmal1* gene.

6. A clock gene expression level regulator comprising an olive extract.

7. The clock gene expression level regulator as set forth in claim 6, wherein the olive extract comprises at least 30% by weight of an olive-derived polyphenol.

8. The clock gene expression level regulator as set forth in claim 7, wherein the olive-derived polyphenol comprises at least one compound selected from the group consisting of oleuropein, 3,4-dihydroxyphenylethanol-elenolic acid, derivatives thereof, and salts of the foregoing.

9. The clock gene expression level regulator as set forth in any one of claims 6 to 8, wherein the olive extract is extracted from olive leaves with water, ethanol, or a mixture thereof.

10. A food or beverage having added thereto the clock gene expression level regulator as set forth in any one of claims 1 to 9.

11. A pharmaceutical composition for preventing, alleviating, or treating indispositions or diseases caused by sleep rhythm disturbance. the pharmaceutical composition comprising at least one compound selected from the group consisting of oleuropein, 3,4-dihydroxyphenylethanol-elenolic acid, derivatives thereof, and salts of the foregoing.

12. A method for preparing a food, beverage or pharmaceutical composition having sleep rhythm-controlling activity, the method comprising the step of incorporating at least one compound selected from the group consisting of oleuropein, 3,4-dihydroxyphenylethanol-elenolic acid, derivatives thereof, and salts of the foregoing.

13. Use of at least one compound selected from the group consisting of oleuropein, 3,4-dihydroxyphenylethanol-elenolic acid, derivatives thereof, and salts of the foregoing, in the preparation of a food, beverage or pharmaceutical composition having sleep rhythm-controlling activity.
